(19) Europäisches Patentamt
European Patent Office
Office européen des brevets

(11) EP 3 305 825 B1

(12) **EUROPEAN PATENT SPECIFICATION**

(45) Date of publication and mention of the grant of the patent:
**16.11.2022 Bulletin 2022/46**

(21) Application number: **16800005.7**

(22) Date of filing: **24.05.2016**

(51) International Patent Classification (IPC):
***C08G 59/24*** (2006.01) ***C08L 63/00*** (2006.01)
***G02B 1/04*** (2006.01) ***G03F 7/038*** (2006.01)

(52) Cooperative Patent Classification (CPC):
**G03F 7/038**

(86) International application number:
**PCT/JP2016/065294**

(87) International publication number:
**WO 2016/190300 (01.12.2016 Gazette 2016/48)**

(54) **PHOTOCURABLE COMPOSITION, CURED PRODUCT AND OPTICAL COMPONENT USING SAME**

LICHTHÄRTBARE ZUSAMMENSETZUNG, GEHÄRTETES PRODUKT UND OPTISCHE KOMPONENTE DAMIT

COMPOSITION PHOTODURCISSABLE, PRODUIT DURCI ET COMPOSANT OPTIQUE L'UTILISANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.05.2015 JP 2015107580**
**13.07.2015 JP 2015139675**

(43) Date of publication of application:
**11.04.2018 Bulletin 2018/15**

(73) Proprietor: **Daicel Corporation**
**Osaka-shi, Osaka 530-0011 (JP)**

(72) Inventors:
- **FUJIKAWA, Takeshi**
**Himeji-shi**
**Hyogo 671-1283 (JP)**
- **ISHIDA, Kyohei**
**Himeji-shi**
**Hyogo 671-1283 (JP)**

(74) Representative: **Grünecker Patent- und Rechtsanwälte**
**PartG mbB**
**Leopoldstraße 4**
**80802 München (DE)**

(56) References cited:
**EP-A1- 3 196 222** **EP-A1- 3 196 679**
**WO-A1-2014/112295** **WO-A1-2015/005210**
**JP-A- 2012 001 689** **JP-A- 2013 095 833**
**JP-A- 2014 196 475** **JP-A- 2014 224 205**
**JP-A- 2015 036 420** **JP-A- 2016 115 779**
**US-A1- 2010 119 835**

- **Robert F Fedors: "A method for estimating both the solubility parameters and molar volumes of liquids", Polymer Engineering and Science, 1 February 1974 (1974-02-01), pages 147-154, XP055572996, DOI: 10.1002/pen.760140211 Retrieved from the Internet: URL:https://www.researchgate.net/profile/D aniel_Delgado/post/How_can_I_calculate_the _molar_volume_of_an_organic_solid_compoun d _at_different_temperatures/attachment/59d6 2c28c49f478072e9dd9a/AS:273542057267206@ 14 42228916496/download/Polymer%20Engineerin g %20&%20Science%20Volume%2014%20issue% 202%2 01974%20&#**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 305 825 B1

## Description

### Technical Field

The present invention relates to photocurable compositions, cured products (cured articles) of the photocurable compositions, and optical components including the cured articles. Specifically, the present invention relates to a photocurable composition which is for use in a UV imprint molding step in the production of an optical component, in which molding (transfer) of the photocurable composition is successively (continuously) performed using a mold through ultraviolet irradiation. The present invention also relates to a cured article and an optical component each obtained using the photocurable composition. This application claims priority to Japanese Patent Application No. 2015-107580, filed on May 27, 2015 to Japan; and Japanese Patent Application No. 2015-139675, filed on July 13, 2015.

Optical components such as a flash lens are now produced by various molding techniques such as injection molding using injection molds. Specifically, such an optical component is obtained by injection-molding a thermoplastic resin such as a polycarbonate, as in Patent Literature (PTL) 1; by injecting an acrylic resin being a low-molecular-weight monomer into a mold, curing and thereby molding the acrylic resin by photoirradiation, as in PTL 2 and PTL 3; by subjecting a silicone resin to liquid injection molding (LIM) as in PTL 4; or by injecting an epoxy resin into a mold and curing the epoxy resin by photoirradiation, as in PTL 5.

### Citation List

### Patent Literature

PTL 1: Japanese Unexamined Patent Application Publication (JP-A) No. 2005-119124
PTL 2: Japanese Patent No. 4124991
PTL 3: JP-A No. H11-092539
PTL 4: Japanese Patent No. 4800383
PTL 5: Japanese Patent No. 3671658

WO 2014/112295 A1 discloses a photocurable composition for nanoimprinting and a method for producing a micropatterned substrate. In particular it discloses a compound that a volume expansion coefficient of 0 to 30% when cured.

US 2010/119835 A1 discloses a clear, low viscosity photocurable composition comprising an epoxy compound, a compound containing an oxetane ring in its molecule, a polyol containing mixture and a cationic photoinitiator and a process for producing opaque three-dimensional articles using said composition in rapid prototyping techniques.

### Summary of the Invention

### Technical Problem

A thermoplastic resin, if used as in PTL 1, requires an extra step for the mounting of the resulting lens (optical component) to an optical device, because the thermoplastic resin lacks reflow resistance, and the lens cannot be subjected to reflow mounting. An acrylic resin, which is a curable material, if used as in PTL 2 and PTL 3, hardly forms a highly precise molded article. A silicone resin, if used in liquid injection molding as in PTL 4, hardly forms a highly precise molded article, although the silicone resin has reflow resistance. In addition, the liquid injection molding requires extra portions of the resin in unnecessary portions, such as a sprue and runners, of the molded article, and this impedes improvements in utilization rate of the resin. The proposed molding technique using an ultravioletcurable resin such as an epoxy resin in PTL 5 is disadvantageous in shape accuracy of the molded article, durability of the mold, and releasability of the molded article from the mold.

Molded articles having complicated, special shapes have been recently required for use as lenses and other optical components so as to have higher functions and higher performance. In particular, cast molding techniques using curable resins can give molded articles having such shapes as not to be achieved by existing techniques such as injection molding and receive significant attention. Among the cast molding techniques, UV imprint molding, which is imprint molding using a mold and curing resins by ultraviolet irradiation, enables not only highly accurate shape transfer, but also repeated use of molds in the production of optical components, and contributes to significantly better productivity. As the molds, silicon molds are widely used because of having shape transferability, mold releasability, and transparency at excellent levels. Disadvantageously, however, the silicon molds gradually swell by the action of resins with an increasing

use frequency and become unable to give cured articles with good accuracy.

Another proposed technique is imprint molding using a resin film. This technique, however, can use the mold only once and is susceptible to improvements in economic efficiency and in environment aspect. Increase in use frequency per one mold can significantly contribute to cost reduction.

Accordingly, the present invention has an object to provide a photocurable composition as follows. This photocurable composition, when cured using a silicon mold by ultraviolet irradiation, has excellent curability, surely offers shape transferability, still less causes the resin-induced swelling of the silicon mold, and allows the silicon mold to have better durability. The present invention has another object to provide an optical component that can have a highly accurate shape even being a complicated, special shape and still has transparency and heat resistance at excellent levels.

Solution to Problem

After intensive investigations to achieve the objects, the inventors of the present invention found that a photocurable composition including a specific cycloaliphatic epoxy compound, an oxetane compound, a glycidyl ether epoxy compound, and a photoinitiator, when cured using a silicon mold by ultraviolet irradiation, offers curability and shape transferability at excellent levels, less causes resin-induced swelling of the mold and allows the mold to have better durability. The present invention has been made on the basis of these findings.

The scope of the invention is as defined by the appended claims land 2 One aspect of the invention is providing a photocurable composition including components (A), (B), (C), and (D), in which the component (A) is present in a content of 10 to 50 weight percent of the totality of photocurable compounds contained in the photocurable composition. The component (A) is a cycloaliphatic epoxy compound represented by Formula (a):

[Chem. 1]

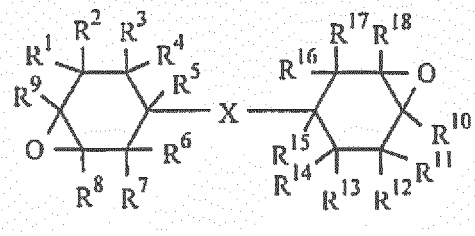

(a)

wherein $R^1$ to $R^{18}$ are each, identically or differently, selected from hydrogen, halogen, a hydrocarbon group optionally containing oxygen or halogen, and optionally substituted alkoxy; and X is selected from a single bond and a linkage group. The component (B) is an oxetane compound having a solubility parameter (SP) of 9.5 $(cal/cm^3)^{1/2}$ or more as determined by the Fedors' method. The component (C) is a glycidyl ether epoxy compound having a molecular weight of 250 or more. The component (D) is a photoinitiator.

In the photocurable composition, the component (B) may be present in a content of 10 to 60 weight percent of the totality of photocurable compounds contained in the photocurable composition.

In the photocurable composition, the component (C) may be present in a content of 10 to 60 weight percent of the totality of photocurable compounds contained in the photocurable composition.

In the photocurable composition, the component (D) may be present in a proportion of 0.05 to 10 parts by weight per 100 parts by weight of the totality of photocurable compounds contained in the photocurable composition.

In the photocurable composition, the component (A) may have a solubility parameter of 9.2 $(cal/cm^3)^{1/2}$ or more as determined by the Fedors' method.

In the photocurable composition, the component (C) may have a solubility parameter of 9.5 $(cal/cm^3)^{1/2}$ or more as determined by the Fedors' method.

The photocurable composition may further include at least one of an antioxidant and an ultraviolet absorber.

The photocurable composition may further include a surface modifier.

The photocurable composition may be used for optical component use.

The present invention also provides a cured article resulting from curing the photocurable composition.

The cured article may be molded using a silicon mold.

The silicon mold used may be made from a polydimethylsiloxane.

The cured article may be cured through photoirradiation using a UV-LED (at a wavelength of 350 to 400 nm).

The present invention also provides an optical component including the cured article.

The optical component may be for use in diffusion of light.

In addition and advantageously, the present invention provides an optical device including the optical component.

Specifically,the present invention relates to a photocurable composition according to appended claims 1 and 2, a cured article according to appended claim 5 and a method for producing a cured article according to appended claim 12.

Advantageous Effects of Invention

The photocurable composition according to the present invention has the configuration and, when cured using a silicon mold by ultraviolet irradiation, offers curability and shape transferability at excellent levels. In addition, the photocurable composition less causes resin-induced swelling of the mold, allows the silicon mold to have better durability and to be usable repeatedly, and thereby also has excellent economic efficiency. The optical component according to the present invention can have a highly accurate shape even being a complicated, special shape and still has transparency and heat resistance at excellent levels.

Description of Embodiments

Component (A)

The photocurable composition according to the present invention includes one or more cycloaliphatic epoxy compounds represented by Formula (a). These cycloaliphatic epoxy compounds are also generically referred to as a "component (A)". Formula (a) is expressed as follows:

[Chem. 2]

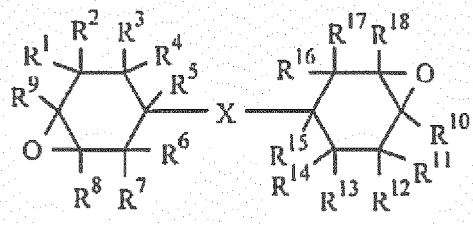

(a)

wherein $R^1$ to $R^{18}$ are each, identically or differently, selected from hydrogen, halogen, a hydrocarbon group optionally containing oxygen or halogen, and optionally substituted alkoxy; and X is selected from a single bond and a linkage group.

Non-limiting examples of the halogen as $R^1$ to $R^{18}$ include fluorine, chlorine, bromine, and iodine.

Examples of the hydrocarbon group as $R^1$ to $R^{18}$ include aliphatic hydrocarbon groups, alicyclic hydrocarbon groups, aromatic hydrocarbon groups, and groups each including two or more of them bonded to each other.

Non-limiting examples of the aliphatic hydrocarbon groups include alkyls containing 1 to 20 carbon atoms (i.e., $C_1$-$C_{20}$ alkyls), such as methyl, ethyl, propyl, isopropyl, butyl, hexyl, octyl, isooctyl, decyl, and dodecyl, of which $C_1$-$C_{10}$ alkyls are preferred, and $C_1$-$C_4$ alkyls are particularly preferred; $C_2$-$C_{20}$ alkenyls such as vinyl, allyl, methallyl, 1-propenyl, isopropenyl, 1-butenyl, 2-butenyl, 3-butenyl, 1-pentenyl, 2-pentenyl, 3-pentenyl, 4-pentenyl, and 5-hexenyl, of which $C_2$-$C_{10}$ alkenyls are preferred, and $C_2$-$C_4$ alkenyls are particularly preferred; and $C_2$-$C_{20}$ alkynyls such as ethynyl and propynyl, of which $C_2$-$C_{10}$ alkynyls are preferred, and $C_2$-$C_4$ alkynyls are particularly preferred.

Non-limiting examples of the alicyclic hydrocarbon groups include $C_3$-$C_{12}$ cycloalkyls such as cyclopropyl, cyclobutyl, cyclopentyl, cyclohexyl, and cyclododecyl; $C_3$-$C_{12}$ cycloalkenyls such as cyclohexenyl; and $C_4$-$C_{15}$ bridged hydrocarbon groups such as bicycloheptyl and bicycloheptenyl.

Non-limiting examples of the aromatic hydrocarbon groups include $C_6$-$C_{14}$ aryls such as phenyl and naphthyl, of which $C_6$-$C_{10}$ aryls are preferred.

Examples of the hydrocarbon group optionally containing oxygen or halogen, as $R^1$ to $R^{18}$, include groups resulting from replacing at least one hydrogen atom in the hydrocarbon groups with an oxygen-containing group or a halogen-containing group. Non-limiting examples of the oxygen-containing group include hydroxy; hydroperoxy; $C_1$-$C_{10}$ alkoxys such as methoxy, ethoxy, propoxy, isopropyloxy, butoxy, and isobutyloxy; $C_2$-$C_{10}$ alkenyloxys such as allyloxy; tolyloxy, naphthyloxy, and other $C_6$-$C_{14}$ aryloxys optionally substituted with at least one substituent selected from $C_1$-$C_{10}$ alkyls, $C_2$-$C_{10}$ alkenyls, halogens, and $C_1$-$C_{10}$ alkoxys; $C_7$-$C_{18}$ aralkyloxys such as benzyloxy and phenethyloxy; $C_1$-$C_{10}$ acyloxys such as acetyloxy, propionyloxy, (meth)acryloyloxy, and benzoyloxy; $C_1$-$C_{10}$ alkoxycarbonyls such as methoxycarbonyl, ethoxycarbonyl, propoxycarbonyl, and butoxycarbonyl; phenoxycarbonyl, tolyloxycarbonyl, naphthyloxycarbonyl, and other $C_6$-$C_{14}$ aryloxycarbonyls optionally substituted with at least one substituent selected from $C_1$-$C_{10}$

alkyls, $C_2$-$C_{10}$ alkenyls, halogens, and $C_1$-$C_{10}$ alkoxys; $C_7$-$C_{18}$ aralkyloxycarbonyls such as benzyloxycarbonyl; epoxy-containing groups such as glycidyloxy; oxetanyl-containing groups such as ethyloxetanyloxy; $C_1$-$C_{10}$ acyls such as acetyl, propionyl, and benzoyl; isocyanato; sulfo; carbamoyl; oxo; and groups each including two or more of these groups bonded to each other through a single bond or a linkage group such as $C_1$-$C_{10}$ alkylene. Non-limiting examples of the halogen-containing group include fluorine, chlorine, bromine, and iodine.

Non-limiting examples of the alkoxy as $R^1$ to $R^{18}$ include $C_1$-$C_{10}$ alkoxys such as methoxy, ethoxy, propoxy, isopropyloxy, butoxy, and isobutyloxy.

Non-limiting examples of the optionally substituted alkoxy include halogens, hydroxy, $C_1$-$C_{10}$ alkoxys, $C_2$-$C_{10}$ alkenyloxys, $C_6$-$C_{14}$ aryloxys, $C_1$-$C_{10}$ acyloxys, mercapto, $C_1$-$C_{10}$ alkylthios, $C_2$-$C_{10}$ alkenylthios, $C_6$-$C_{14}$ arylthios, $C_1$-$C_{18}$ aralkylthios, carboxy, $C_1$-$C_{10}$ alkoxycarbonyls, $C_6$-$C_{14}$ aryloxycarbonyls, $C_7$-$C_{18}$ aralkyloxycarbonyls, amino, mono- or di-($C_1$-$C_{10}$ alkyl) aminos, $C_1$-$C_{10}$ acylaminos, epoxy-containing groups, oxetanyl-containing groups, $C_1$-$C_{10}$ acyls, oxo, and groups each including two or more of these groups bonded to each other through a single bond or a linkage group such as $C_1$-$C_{10}$ alkylene.

In particular, $R^1$ to $R^{18}$ are preferably hydrogens.

X in Formula (a) is selected from a single bond and a linkage group (divalent group containing one or more atoms). Non-limiting examples of the linkage group include divalent hydrocarbon groups, alkenylenes with part or all of carbon-carbon double bond(s) being epoxidized, carbonyl, ether bond, ester bond, amido, and groups each including two or more of these groups linked to each other.

Non-limiting examples of the divalent hydrocarbon groups include linear or branched $C_1$-$C_{18}$ alkylenes such as methylene, methylmethylene, dimethylmethylene, ethylene, propylene, and trimethylene, of which linear or branched $C_1$-$C_3$ alkylenes are preferred; and $C_3$-$C_{12}$ cycloalkylenes and $C_3$-$C_{12}$ cycloalkylidenes, such as 1,2-cyclopentylene, 1,3-cyclopentylene, cyclopentylidene, 1,2-cyclohexylene, 1,3-cyclohexylene, 1,4-cyclohexylene, and cyclohexylidene, of which $C_3$-$C_6$ cycloalkylenes and $C_3$-$C_6$ cycloalkylidenes are preferred.

The alkenylenes with part or all of carbon-carbon double bond(s) being epoxidized are hereinafter also referred to as "epoxidized alkenylenes". Non-limiting examples of the alkenylenes from which the epoxidized alkenylenes are derived include $C_2$-$C_8$ linear or branched alkenylenes such as vinylene, propenylene, 1-butenylene, 2-butenylene, butadienylene, pentenylene, hexenylene, heptenylene, and octenylene. In particular, the epoxidized alkenylenes are preferably selected from alkenylenes with all of carbon-carbon double bond(s) being epoxidized, and are more preferably selected from $C_2$-$C_4$ alkenylenes with all of carbon-carbon double bond(s) being epoxidized.

Representative, but non-limiting examples of the compounds represented by Formula (a) include 3,4-epoxycyclohexylmethyl (3,4-epoxy)cyclohexanecarboxylate, (3,4,3',4'-diepoxy)bicyclohexyl, bis(3,4-epoxycyclohexylmethyl) ether, 1,2-epoxy-1,2-bis(3,4-epoxycyclohex-1-yl)ethane, 2,2-bis(3,4-epoxycyclohex-1-yl)propane, and 1,2-bis(3,4-epoxycyclohex-1-yl)ethane. Among them, 3,4-epoxycyclohexylmethyl (3,4-epoxy)cyclohexanecarboxylate and (3,4,3',4'-diepoxy)bicyclohexyl are particularly preferred. The compounds represented by Formula (a) are also available as a commercial product typically under the trade name of CELLOXIDE 2021P (from Daicel Corporation). The photocurable composition may include each of these compounds alone or in combination.

The compounds represented by Formula (a) can each be produced typically by reacting a compound represented by Formula (a') with a peroxy acid (such as peracetic acid) to epoxidize the double bonds in Formula (a'). $R^1$ to $R^{18}$ and X in Formula (a') are as defined above.

[Chem. 3]

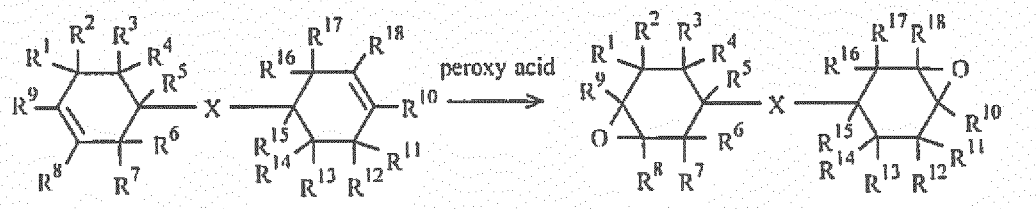


(a') (a)

The component (A) in the photocurable composition according to the present invention is present in a content of 10 to 50 weight percent, preferably 10 to 45 weight percent, more preferably 10 to 40 weight percent, and furthermore preferably 10 to 35 weight percent, of the totality (total amount; 100 weight percent) of photocurable compounds contained in the photocurable composition. When two or more different compounds are contained as the component (A), the term "content" refers to the total content of them. The component (A), as being present in a content within the range, allows

the photocurable composition to offer excellent curability and to readily give a cured article having transparency and heat resistance at excellent levels. The "photocurable compounds" include the component (A), the component (B), the component (C), and one or more other photocurable compounds, which are optional components.

The component (A) has a solubility parameter (SP) of typically 9.2 or more, preferably 9.5 to 15, more preferably 9.6 to 14, and furthermore preferably 9.7 to 13, as determined at 25°C by the Fedors' method. For the solubility parameter, see Polym. Eng. Sci., 14, 147(1974). The component (A), when having a solubility parameter within the range, allows the curable composition to less cause the swelling of the mold, thereby allows the mold to maintain satisfactory durability and to be usable in transfer a larger number of times. The solubility parameter is expressed in $(cal/cm^3)^{1/2}$ and can be calculated typically by the method described in the working examples.

Component (B)

The photocurable composition according to the present invention includes one or more oxetane compounds having a solubility parameter of 9.5 $(cal/cm^3)^{1/2}$ or more as determined at 25°C by the Fedors' method. These oxetane compounds are hereinafter generically referred to as a "components (B)". The compounds as the component (B) are represented typically by Formula (b):

[Chem. 4]

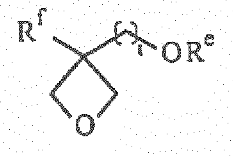

(b)

wherein $R^e$ represents a monovalent organic group; $R^f$ is selected from hydrogen and ethyl; and t represents an integer of 0 or more.

Examples of the monovalent organic group as $R^e$ include monovalent hydrocarbon groups, monovalent heterocyclic groups, substituted oxycarbonyls (such as alkoxycarbonyls, aryloxycarbonyls, aralkyloxycarbonyls, and cycloalkyloxycarbonyls), substituted carbamoyls (such as N-alkylcarbamoyls and N-arylcarbamoyls), acyls (exemplified by aliphatic acyls such as acetyl; and aromatic acyls such as benzoyl), and monovalent groups each including two or more of these groups bonded to each other through a single bond or a linkage group.

Examples of the monovalent hydrocarbon groups are as with $R^1$ to $R^{18}$ in Formula (a) .

The monovalent hydrocarbon groups may each have one or more substituents. Non-limiting examples of the substituents include halogens, oxo, hydroxy, substituted oxys (such as alkoxys, aryloxys, aralkyloxys, and acyloxys), carboxy, substituted oxycarbonyls (such as alkoxycarbonyls, aryloxycarbonyls, and aralkyloxycarbonyls), substituted or unsubstituted carbamoyls, cyano, nitro, substituted or unsubstituted aminos, sulfo, and heterocyclic groups. The hydroxy and carboxy may each be protected with a protecting group commonly used in the field of organic synthesis.

Heterocycles constituting the heterocyclic groups include aromatic heterocycles and non-aromatic heterocycles. Examples of the heterocycles include, but are not limited to, oxygen-containing heterocycles, sulfur-containing heterocycles, and nitrogen-containing heterocycles, where the oxygen, sulfur, and nitrogen are contained as heteroatoms. Non-limiting examples of the oxygen-containing heterocycles include 4-membered rings such as oxetane ring; 5-membered rings such as furan, tetrahydrofuran, oxazole, isoxazole, and y-butyrolactone rings; 6-membered rings such as 4-oxo-4H-pyran, tetrahydropyran, and morpholine rings; fused rings such as benzofuran, isobenzofuran, 4-oxo-4H-chromene, chroman, and isochroman rings; and bridged rings such as 3-oxatricyclo[$4.3.1.1^{4,8}$]undecan-2-one, and 3-oxatricyclo[$4.2.1.0^{4,8}$]nonan-2-one rings. Non-limiting examples of the sulfur-containing heterocycles include 5-membered rings such as thiophene, thiazole, isothiazole, and thiadiazole rings; 6-membered rings such as 4-oxo-4H-thiopyran ring; and fused rings such as benzothiophene ring. Non-limiting examples of the nitrogen-containing heterocycles include 5-membered rings such as pyrrole, pyrrolidine, pyrazole, imidazole, and triazole rings; 6-membered rings such as pyridine, pyridazine, pyrimidine, pyrazine, piperidine, and piperazine rings; and fused rings such as indole, indoline, quinoline, acridine, naphthyridine, quinazoline, and purine rings. Non-limiting examples of the monovalent heterocyclic groups include groups resulting from removing one hydrogen atom each from the structural formulae of the heterocycles.

The heterocyclic groups may each have one or more substituents. Examples of the substituents include the substituents which the hydrocarbon groups may have; as well as alkyls (such as methyl, ethyl, and other $C_1$-$C_4$ alkyls), $C_3$-$C_{12}$ cycloalkyls, and $C_6$-$C_{14}$ aryls (such as phenyl and naphthyl).

Non-limiting examples of the linkage group include carbonyl (-CO-), ether bond (-O-), thioether bond (-S-), ester bond (-COO-), amido bond (-CONH-), carbonate bond (-OCOO-), silyl bond (-Si-), and groups each including two or

more of these linked to each other.

The repetition number t represents an integer of 0 or more and is typically 0 to 12, and preferably 1 to 6.

Non-limiting examples of the compounds represented by Formula (b) include compounds represented by Formulae (b-1) to (b-4):

[Chem. 5]

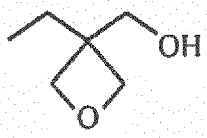

(b-1)

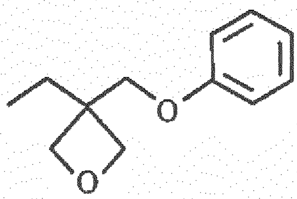

(b-2)

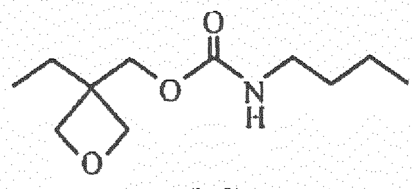

(b-3)

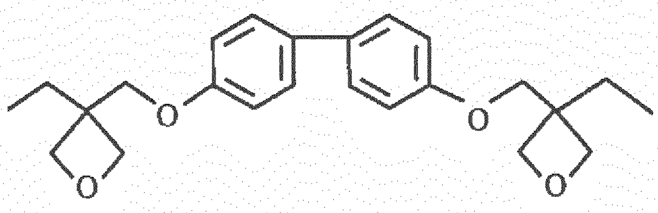

(b-4)

Non-limiting examples of commercial products usable as the compounds represented by Formula (b) include those available under the trade names of ARON OXETANE OXT-101 and ARON OXETANE OXT-610 (from Toagosei Co., Ltd.); and the trade name of OXBP (from Ube Industries, Ltd.).

The component (B) in the photocurable composition according to the present invention is present in a content of typically 10 to 60 weight percent, preferably 10 to 50 weight percent, more preferably 15 to 45 weight percent, and furthermore preferably 15 to 35 weight percent, of the totality (100 weight percent) of photocurable compounds contained in the photocurable composition. When two or more different compounds are contained as the component (B), the term "content" refers to the total content of them. The component (B), when being present in a content within the range, allows the photocurable composition to offer excellent curability. The "photocurable compounds" include the component (A), the component (B), the component (C), and one or more other photocurable compounds, which are optional components.

The component (B) has a solubility parameter of 9.5 or more, preferably 9.6 to 15, more preferably 9.7 to 14, and furthermore preferably 9.8 to 13, as determined at 25°C by the Fedors' method. For the solubility parameter, see Polym. Eng. Sci., 14, 147(1974). The component (B), as having a solubility parameter within the range, allows the photocurable composition to less cause the swelling of the mold and allows the mold to maintain satisfactory durability and to be usable in transfer a larger number of times. The solubility parameter is expressed in $(cal/cm^3)^{1/2}$ and can be calculated typically by the method described in the working examples.

Component (C)

The photocurable composition according to the present invention contains one or more glycidyl ether epoxy compounds having a molecular weight of 250 or more. These glycidyl ether epoxy compounds are also generically referred to as a "component (C)". Examples of the glycidyl ether epoxy compounds include aromatic glycidyl ether epoxy compounds, alicyclic glycidyl ether epoxy compounds, and aliphatic glycidyl ether epoxy compounds.

Examples of the aromatic glycidyl ether epoxy compounds include, but are not limited to, bisphenol-A diglycidyl ethers and bisphenol-F diglycidyl ethers. Non-limiting examples of the bisphenol-A diglycidyl ethers include bisphenol-A bis(propylene glycol glycidyl ether) ether and bisphenol-A bis(triethylene glycol glycidyl ether) ether. Non-limiting examples of commercial products usable as the bisphenol-A diglycidyl ethers include those available under the trade names of Rikaresin BPO-20E and Rikaresin BPO-60E (from New Japan Chemical Co., Ltd.).

Non-limiting examples of the aliphatic glycidyl ether epoxy compounds include, but are not limited to, ethylene glycol diglycidyl ether, diethylene glycol diglycidyl ether, and triethylene glycol diglycidyl ethers. Non-limiting examples of commercial products usable as the aliphatic glycidyl ether epoxy compounds include those available under the trade

names of EPOLIGHT 40E, EPOLIGHT 100E, EPOLIGHT 200E, and EPOLIGHT 400E (from Kyoeisha Chemical Co., Ltd.); and the trade name of YH-300 (from NIPPON STEEL & SUMIKIN CHEMICAL CO., LTD.)

The component (C) has a molecular weight of 250 or more (typically from 250 to less than 2000), preferably 300 to 1500, more preferably 350 to 1300, furthermore preferably 400 to 1000, and particularly preferably 400 to 600. The component (C), as having a molecular weight within the range, allows the photocurable composition to have excellent shape transferability. The component (C), if having an excessively high molecular weight, tends to be difficult to use because of higher viscosity. In contrast, the component (C), if having an excessively low molecular weight, tends to cause the photocurable composition to have lower shape transferability.

The component (C) has a weight average molecular weight (Mw) of typically from 250 to less than 2000, preferably 300 to 1800, more preferably 350 to 1500, furthermore preferably 400 to 1300, and particularly preferably 400 to 1000, as determined by GPC and calibrated with a polystyrene standard.

The component (C) in the photocurable composition according to the present invention is present in a content of typically 10 to 60 weight percent, preferably 10 to 50 weight percent, more preferably 15 to 45 weight percent, and furthermore preferably 20 to 35 weight percent, of the totality (100 weight percent) of photocurable compounds contained in the photocurable composition. When two or more different compounds are contained as the component (C), the term "content" refers to the total content of them. The component (C), when being present in a content within the range, may allow the photocurable composition to offer excellent curability. The "photocurable compounds" include the component (A), the component (B), the component (C), and one or more other photocurable compounds, which are optional components.

The component (C) has a solubility parameter of typically 9.5 or more, preferably 9.6 to 15, more preferably 9.7 to 14, and furthermore preferably 9.8 to 13, as determined at 25°C by the Fedors' method. For the solubility parameter, see Polym. Eng. Sci., 14, 147(1974). The component (C), when having a solubility parameter within the range, may allow the photocurable composition to less cause the swelling of the mold and allows the mold to maintain satisfactory durability and to be usable in transfer a larger number of times. The solubility parameter is expressed in $(cal/cm^3)^{1/2}$ and can be calculated typically by the method described in the working examples.

Other Photocurable Compounds

In addition to the components (A), (B), and (C), the photocurable composition according to the present invention may further include one or more other photocurable compounds as needed within the ranges not adversely affecting the advantageous effects of the present invention. Non-limiting examples of the other photocurable compounds include epoxy compounds other than the component (A) and the component (C); oxetane compounds other than the component (B); vinyl ether compounds; acrylic compounds; and silicone compounds. The other photocurable compound or compounds may be present in a content of typically 30 weight percent or less, preferably 20 weight percent or less, and more preferably 10 weight percent or less, of the totality (100 weight percent) of all photocurable compounds. The other photocurable compound(s), if present in a content greater than the range, tends to cause the photocurable composition to less satisfactorily offer the advantageous effects of the present invention.

Component (D)

The photocurable composition according to the present invention contains one or more photoinitiators. These photoinitiators are also generically referred to as a "component (D)". Examples of the component (D) for use herein include known or common ones that can initiate cationic polymerization or anion polymerization, such as cationic photoinitiators, anionic photoinitiators, and photoacid generators.

Non-limiting examples of the cationic photoinitiators include borate anions $[(Y)_kB(Phf)_{4-k}]^-$, where Y is selected from a $C_6$-$C_{30}$ aromatic hydrocarbon group and a $C_4$-$C_{30}$ heterocyclic group each of which may have one or more substituents (excluding halogen-containing groups); Phf represents a phenyl group with at least one of hydrogen atoms being substituted with at least one selected from perfluoroalkyls, perfluoroalkoxys, and halogens; and k represents an integer of 0 to 3.

Non-limiting examples of the component (D) include sulfonium salts such as triarylsulfonium hexafluorophosphates and triarylsulfonium hexafluoroantimonates; iodonium salts such as diaryliodonium hexafluorophosphates, diphenyliodonium hexafluoroantimonate, bis(dodecylphenyl)iodonium tetrakis(pentafluorophenyl)borate, and iodonium [4-(4-methylphenyl-2-methylpropyl)phenyl]hexafluorophosphate; phosphonium salts such as tetrafluorophosphonium hexafluorophosphate; and pyridinium salts. Non-limiting examples of the commercial products suable as the component (D), which is a photoinitiator, include those available under the trade names of CPI-100P, CPI-101A, and CPI-200K (from San-Apro Ltd.).

The component (D) in the photocurable composition according to the present invention is present in a proportion of typically 0.05 to 10 parts by weight, preferably 0.1 to 5 parts by weight, more preferably 0.1 to 3 parts by weight, and

furthermore preferably 0.1 to 1 part by weight, per 100 parts by weight of the totality of photocurable compounds contained in the photocurable composition. When two or more photoinitiators are contained as the component (D), the term "content" refers to the total content of them. The component (D), when being present in a content within the range, may allow the photocurable composition to offer excellent curability. The "photocurable compounds" include all the component (A), the component (B), the component (C), and the other photocurable compounds, which are optional components.

Other Components

The photocurable composition according to the present invention may further contain one or more other components in addition to the components (A), (B), (C), and (D), within the ranges not adversely affecting the advantageous effects of the present invention. Non-limiting examples of the other components include antioxidants, ultraviolet absorbers, surface modifiers, fillers, photosensitizers, antifoaming agents, leveling agents, coupling agents, surfactants, flame retardants, decolorizing agents, adhesion imparting agents, and colorants. The photocurable composition may contain each of them alone or in combination.

The photocurable composition according to the present invention preferably contains, among them, at least one of an antioxidant and a ultraviolet absorber. This is preferred for effectively contributing to better thermal yellowing resistance of the cured article. Non-limiting examples of the antioxidants include phenolic antioxidants, phosphorus antioxidants, thio ester antioxidants, and amine antioxidants. The photocurable composition may contain each of them alone or in combination. Among them, phenolic antioxidants are preferred in the present invention, because they particularly effectively allow the photocurable composition to have better thermal yellowing resistance.

Non-limiting examples of the phenolic antioxidants include pentaerythritol tetrakis[3-(3,5-di-t-butyl-4-hydroxyphenyl)propionate], thiodiethylene bis[3-(3,5-di-t-butyl-4-hydroxyphenyl)propionate], octadecyl 3-(3,5-di-t-butyl-4-hydroxyphenyl)propanoate, N,N'-hexamethylenebis[3-(3,5-di-t-butyl-4-hydroxyphenyl)propionamide], octyl 3-(4-hydroxy-3,5-diisopropylphenyl)propionate, 1,3,5-tris(4-hydroxy-3,5-di-t-butylbenzyl)-2,4,6-trimethylbenzene, 2,4-bis(dodecylthiomethyl)-6-methylphenol, and calcium bis[3,5-di(t-butyl)-4-hydroxybenzyl(ethoxy)phosphinate]. Non-limiting examples of commercial products usable as the phenolic antioxidants include those available under the trade names of Irganox 1010, Irganox 1035, Irganox 1076, Irganox 1098, Irganox 1135, Irganox 1330, Irganox 1726, and Irganox 1425WL (from BASF SE).

The antioxidant may be used in an amount of typically 0.05 to 5 parts by weight, and preferably 0.1 to 3 parts by weight, per 100 parts by weight of the totality of photocurable compounds contained in the photocurable composition. The antioxidant, when used in an amount within the range, may protect the cured article from yellowing.

Non-limiting examples of the ultraviolet absorber include benzophenone ultraviolet absorbers, benzotriazole ultraviolet absorbers, and benzoate ultraviolet absorbers, of which benzophenone ultraviolet absorbers and benzotriazole ultraviolet absorbers are preferred. Non-limiting examples of the commercial products usable as the benzotriazole ultraviolet absorbers include those available under the trade names of KEMISORB 71, KEMISORB 73, KEMISORB 74, and KEMISORB 79 (from Chemipro Kasei Kaisha, Ltd.).

The ultraviolet absorber may be used in an amount of typically 0.01 to 3 parts by weight, and preferably 0.02 to 1 part by weight, per 100 parts by weight of the totality of photocurable compounds contained in the photocurable composition. The ultraviolet absorber, when used in an amount within the range, may protect the cured article from deterioration by ultraviolet rays and from coloring.

The photocurable composition according to the present invention also preferably contains a surface modifier. This is preferred for better wettability with the silicon mold and for less causing bubble entrainment upon coating. Examples of the surface modifier include compounds used typically as or for surfactants and leveling agents. Non-limiting examples of the surfactants and the leveling agents include silicone surfactants, acrylic surfactants, and fluorine-containing surfactants. Non-limiting examples of the silicone surfactants include polyether-modified polydimethylsiloxanes. Non-limiting examples of commercial products usable as the surfactants and the leveling agents include those available under the trade names of BYK-333 and BYK-345 (from BYK Japan KK).

The surface modifier may be used in an amount of typically 0.01 to 3 parts by weight, and preferably 0.03 to 1 part by weight, per 100 parts by weight of the totality of photocurable compounds contained in the photocurable composition. The surface modifier, when used in an amount within the range, can restrain bubble entrainment upon coating, without adversely affecting the properties of the cured article.

The photocurable composition according to the present invention may contain one or more fillers having various functions. Non-limiting examples of the functions include insulating properties, strength (strengthening function), viscosity, flame retardancy, conductivity, brightness, and antimicrobic activity. The fillers include inorganic fillers and organic fillers.

Non-limiting examples of the inorganic fillers include calcium carbonate, magnesium carbonate, clay, kaolin, calcium phosphate, hydroxyapatite, mica, talc, silica, quartz powder, glass powder, diatomaceous earth, nepheline syenite, cristobalite, wollastonite, aluminum hydroxide, iron oxide, zinc oxide, titanium oxide, alumina, calcium sulfate, barium sulfate, dolomite, silicon carbide, silicon nitride, boron nitride, metal powders, graphite, carbon black, hydroxyapatite/silver, and zeolite/silver. Non-limiting examples of the organic fillers include particulates of polymers such as crosslinked poly(methyl methacrylate)s. The photocurable composition may contain each of them alone or in combination.

The filler may be treated on its surface typically with a surface-treatment agent such as a silane coupling agent.

The filler is not limited in shape and may have any shape such as a spherical, ellipsoidal, cylindrical, or prismatic shape. The particle size of the filer can be selected as appropriate according to the intended use, within a range not adversely affecting dispersibility. For example, the filler may have a diameter or major axis of typically about 0.001 to about 50 μm.

The filler particles may be used in an amount of typically 1 to 30 parts by weight, and preferably 5 to 20 parts by weight, per 100 parts by weight of the totality of photocurable compounds contained in the photocurable composition.

The photocurable composition according to the present invention may contain a colorant. The colorant (or coloring agent) includes pigments and dyes. The photocurable composition may contain each of them alone or in combination.

Non-limiting examples of the pigments include inorganic pigments; organic pigments; and pigments resulting from coating such inorganic pigments with organic materials such as resins. Non-limiting examples of the inorganic pigments include carbon black, chromium oxide, iron oxide, black titanium oxide, acetylene black, lampblack, bone black, graphite, iron black, copper chrome black, copper iron manganese black, chromium cobalt iron black, ruthenium oxide, graphite, fine particles of metals (such as aluminum), fine particles of metal oxides, fine particles of complex oxides, fine particles of metal sulfides, and fine particles of metal nitrides. Non-limiting examples of the organic pigments include perylene black, cyanine black, aniline black, azo pigments, anthraquinone pigments, isoindolinone pigments, indanthrene pigments, indigo pigments, quinacridone pigments, dioxazine pigments, tetraazaporphyrin pigments, triarylmethane pigments, phthalocyanine pigments, perylene pigments, benzimidazolone pigments, and rhodamine pigments.

Non-limiting examples of the dyes include azo dyes, anthraquinone dyes, indigo dyes, carbonyl dyes, xanthene dyes, quinonimine dyes, quinoline dyes, tetraazaporphyrin dyes, triarylmethane dyes, naphthoquinone dyes, nitro dyes, phthalocyanine dyes, fluoran dyes, perylene dyes, methine dyes, and rhodamine dyes, where the anthraquinone dyes are exemplified typically by Acid Violet 39, Acid Violet 41, Acid Violet 42, Acid Violet 43, Acid Violet 48, Acid Violet 51, Acid Violet 34, Acid Violet 47, Acid Violet 109, Acid Violet 126, Basic Violet 24, Basic Violet 25, Disperse Violet 1, Disperse Violet 4, Disperse Violet 26, Disperse Violet 27, Disperse Violet 28, Disperse Violet 57, Solvent Violet 11, Solvent Violet 13, Solvent Violet 14, Solvent Violet 26, Solvent Violet 28, Solvent Violet 31, Solvent Violet 36, Solvent Violet 37, Solvent Violet 38, Solvent Violet 48, Solvent Violet 59, Solvent Violet 60, Vat Violet 13, Vat Violet 15, and Vat Violet 16.

The content of the colorant can be adjusted as appropriate according to the intended use and is typically about 10 to about 300 ppm of the totality of the photocurable composition according to the present invention. The lower limit of the content is preferably 50 ppm, and particularly preferably 100 ppm. When two or more different colorants are contained, the term "content" refers to the total amount of them.

The photocurable composition according to the present invention has a viscosity (25°C) of typically 100 to 10000 mPa·s, and preferably 100 to 5000 mPa·s. The photocurable composition, when having a viscosity within the range, has excellent shape transferability. The viscosity can be measured with any of various viscometers.

Cured Article

The cured article according to the present invention can be produced typically by a method including steps 1 and 2 as follows.

The step 1 is the step of charging the photocurable composition according to the present invention into an optical-component mold (mold for the formation of an optical component).

The step 2 is the step of irradiating the curable composition with light to cure the curable composition.

In the step 1, the photocurable composition may be charged into the optical-component mold typically through cast molding or injection molding. The photocurable composition, when to be charged through cast molding, can be charged by bringing the photocurable composition into contact with the optical-component mold. The photocurable composition, when to be charged through injection molding, can be charged by injecting the photocurable composition into the optical-component mold. Examples of the material constituting the optical-component mold include, but are not limited to, metals, glass, plastics, and silicon. Among them, the optical-component mold is preferably a silicon mold because of shape transferability, mold releasability, and transparency at excellent levels. Of such silicon molds, preferred is a silicon mold made from a polydimethylsiloxane.

When a mold for Fresnel lens is used as the optical-component mold, the method gives a Fresnel lens as the cured article.

The photoirradiation in the step 2 can be performed by applying light from a light source so that the integral irradiance be in the range typically of 500 to 5000 mJ/cm$^2$, where the light source is exemplified typically by mercury lamps, xenon lamps, carbon arc lamps, metal halide lamps, sunlight, electron beam sources, laser sources, and LED

light sources. Among them, the light source is preferably a UV-LED (at a wavelength of 350 to 400 nm). The irradiance of light may be any irradiance, as long as capable of curing the photocurable composition, but is typically about 500 to about 3000 mJ/cm$^2$.

In an embodiment, the step 2 may further include annealing treatment after the photoirradiation. The annealing treatment may be performed by heating at a temperature of typically 100°C to 200°C for about 30 minutes to about one hour. The annealing treatment may be performed without, or after, demolding from the optical-component mold. The cured article according to the present invention has excellent heat resistance and can satisfactorily retain its shape even in a high-temperature environment of about 100°C to about 200°C. The cured article therefore does not suffer from deviation in pitch even when being subjected to the annealing treatment after demolding from the optical-component mold. This enables efficient production of optical components, where, when the optical components (cured articles) are lenses, the resulting lenses have excellent lens-center alignment accuracy.

In another embodiment, simultaneous molding is performed in the step 1 using an optical-component mold having two or more lens patterns. In this embodiment, the step 2 gives linked lenses including two or more lenses linked to each other. In this case, lenses as the cured articles can be obtained by cutting the linked lenses and removing extra (unnecessary) portions as needed. In the lens mold, the two or more lens patterns may be disposed regularly (arrayed regularly), or disposed at random.

The cured article according to the present invention has a 5% weight loss temperature ($T_{d5}$) of typically 350°C or higher (350°C to 500°C), preferably 380°C or higher, and more preferably 400°C or higher, as measured at a rate of temperature rise of 10°C/min (in the air). The photocurable composition, when having a 5% weight loss temperature within the range, can maintain satisfactory heat resistance. The 5% weight loss temperature can be measured typically by simultaneous thermogravimetry/differential thermal analysis (TG/DTA) .

The cured article according to the present invention has an activation energy of thermal decomposition reaction in the air of typically 150 kJ/mol or more (150 to 350 kJ/mol), preferably 180 kJ/mol or more, and more preferably 200 kJ/mol or more. The photocurable composition, when having an activation energy within the range, can maintain satisfactory heat resistance. The activation energy can be calculated typically by the Ozawa method. The Ozawa method is a method in which thermogravimetry (TG) is performed at three or more different rates of temperature rise to give thermal weight loss data, from which the activation energy of thermal decomposition reaction is calculated.

The cured article according to the present invention has a transmittance of typically 80% or more, preferably 85% or more, and more preferably 90% or more. The cured article, when having a transmittance within the range, can maintain satisfactory transparency. The transmittance is the transmittance typically at a wavelength of 400 nm and can be measured with any of various transmissometers.

The cured article according to the present invention has a linear expansion coefficient ($\alpha 1$) at a temperature equal to or lower than the glass transition temperature of typically 40 to 100 ppm/°C, and preferably 40 to 90 ppm/°C. The cured article according to the present invention has a linear expansion coefficient ($\alpha 2$) at a temperature equal to or higher than the glass transition temperature of typically 90 to 150 ppm/°C, and preferably 90 to 130 ppm/°C. The linear expansion coefficients $\alpha 1$ and $\alpha 2$ of the photocurable composition can be measured typically by TMA.

The cured article according to the present invention has a storage modulus of typically 0.1 GPa or more, and preferably 1 GPa or more, at 25°C. The storage modulus (at 25°C) of the cured article can be measured typically by dynamic viscoelastic measurement.

The cured article according to the present invention has a refractive index of typically 1.40 to 1.60, and preferably 1.48 to 1.58. The cured article according to the present invention has an Abbe number of typically 40 or more, and preferably 50 or more.

Optical Component

The optical component according to the present invention includes the cured article resulting from curing the photocurable composition according to the present invention. The optical component according to the present invention is preferably a lens for use in diffusion or condensation of light, and is, of such lenses, particularly preferably a Fresnel lens. The optical component according to the present invention has sufficient heat resistance for board assembly (mounting) by a high-temperature heat treatment (such as reflow soldering and other high-temperature treatments performed at 260°C or higher). Accordingly, the optical device including (equipped with) the optical component according to the present invention does not require an extra step for mounting the optical component, such as a Fresnel lens, but enables board assembly of the optical component, such as a Fresnel lens, collectively with other components by a high-temperature heat treatment (such as reflow soldering), and can be produced efficiently and inexpensively.

The "Fresnel lens" is a lens including triangular prisms, where the prisms define a sawtooth-profile surface of the lens, and each prism includes a lens surface and a non-lens surface facing the lens surface, as described typically in JP-A No. 2014-38349, JP-A No. 2012-128106, JP-A No. 2013-137442, JP-A No. H04-127101, JP-A No. 2002-264140, Japanese Patent No. 2610029, JP-A No. H09-141663, and JP-A No. H06-11769. The angle $\theta$ formed between the lens

surface and a reference plane continuously decreases (or increases) toward the center. When the lens surfaces alone are viewed continuously, the Fresnel lens forms one convex lens or one concave lens.

Optical Device

The optical device according to the present invention is a device including the optical component according to the present invention. Non-limiting examples of the optical device include optical devices for use typically in cellular phones, smartphones, tablet personal computers, and other portable electronic devices; as well as in near-infrared sensors, millimeter-wave radars, LED spotlighting devices, near-infrared LED lighting systems, mirror monitors, meter panels, head-mounted display (projection type) combiners, head-up display combiners, and other on-vehicle electronic devices.

Examples

Example 8 is in accordance with claim 1,Examples 2 and 3 are in accordance with claim 2. Examples 1, and 4 to 7 are comparative examples and do not fall under the scope of the invention.

The present invention will be illustrated in further detail with reference to several examples below. It should be noted, however, that the examples are by no means intended to limit the scope of the present invention. In Table 1, the proportions of a cycloaliphatic epoxy compound, an oxetane compound, and a glycidyl ether epoxy compound are the proportions (in weight percent) of the individual components relative to the totality of these photocurable compounds; the proportions of a photoinitiator, an antioxidant, an ultraviolet absorber, and an additive are proportions (in part by weight) of the individual component per 100 parts by weight of the totality of the photocurable compounds; and the solubility parameter is expressed in $(cal/cm^3)^{1/2}$

Preparation Example 1: Synthesis of (3,4,3',4'diepoxy)bicyclohexyl (a-1)

A dehydration catalyst was prepared by mixing, with stirring, 70 g (0.68 mol) of 95 weight percent sulfuric acid and 55 g (0.36 mol) of 1,8-diazabicyclo[5.4.0]undecene-7 (DBU) .

A 3-L flask was charged with 1000 g (5.05 mol) of hydrogenated biphenol (4,4'-dihydroxybicyclohexyl), 125 g (0.68 mol in terms of sulfuric acid) of the above-prepared dehydration catalyst, and 1500 g of pseudocumene, and was heated, where the flask was equipped with a stirrer, a thermometer, and a distillation pipe being packed with a dehydrating agent and thermally insulated. Water production was observed from around the time point when the internal temperature exceeded 115°C. The heating (temperature rise) was further continued up to the boiling point of pseudocumene (to an internal temperature of 162°C to 170°C), and dehydration reaction was performed under normal atmospheric pressure. The by-produced water was distilled and discharged through the dehydration tube out of the system. The dehydration catalyst was liquid and was finely dispersed in the reaction mixture under the reaction conditions. Approximately the stoichiometric amount (180 g) of water was distilled after a lapse of 3 hours, and this was defined as reaction completion. The reaction mixture after reaction completion was subjected to distillation using an Oldershaw distilling column including 10 plates to distill off pseudocumene, was further subjected to distillation at an internal temperature of 137°C to 140°C and an internal pressure of 10 Torr (1.33 kPa), and yielded 731 g of bicyclohexyl-3,3'-diene.

Into a reactor, 243 g of the prepared bicyclohexyl-3,3'-diene and 730 g of ethyl acetate were charged. To the mixture, 274 g of a 30 weight percent solution (moisture content: 0.41 weight percent) of peracetic acid in ethyl acetate was added dropwise over about 3 hours while blowing nitrogen into the gas phase portion and controlling the temperature in the reaction system at 37.5°C.

After the completion of the dropwise addition of the peracetic acid solution in ethyl acetate, the resulting mixture was aged at 40°C for one hour, and the reaction was completed. The crude mixture upon the completion of the reaction was washed with water at 30°C, from which low-boiling compounds were removed at 70°C and 20 mmHg, and yielded 270 g of a reaction product. The reaction product had an oxirane oxygen content of 15.0 weight percent.

The reaction product was also subjected to $^1$H-NMR measurement to find that a peak at a $\delta$ of about 4.5 to about 5 ppm disappeared, where this peak is assigned to an internal double bond; but a peak at a $\delta$ of about 3.1 ppm appeared, where this peak is assigned to an epoxy-derived proton. Thus, the reaction product was identified as (3,4,3',4'-diepoxy)bicyclohexyl.

Preparation Example 2: Synthesis of bis(3,4-epoxycyclohexylmethyl) ether (a-2)

In a 5-L reactor, 499 g (12.48 mol) of (granular) sodium hydroxide and 727 mL of toluene were placed. After nitrogen purging, a solution of 420 g (3.74 mol) of tetrahydrobenzyl alcohol in 484 mL of toluene was added, followed by aging at 70°C for 1.5 hours. Next, the mixture was combined with 419 g (2.20 mol) of tetrahydrobenzyl methanesulfonate, aged under reflux for 3 hours, cooled down to room temperature, combined with 1248 g of water to stop the reaction, and the reaction mixture was separated.

The separated organic layer was concentrated, subjected to distillation under reduced pressure, and yielded di-tetrahydrobenzyl ether as a colorless, transparent liquid (in a yield of 85%). The $^1$H-NMR spectrum of the prepared di-tetrahydrobenzyl ether was measured as follows:

$^1$H-NMR ($CDCl_3$): δ 1.23-1.33 (m, 2H), 1.68-1.94 (m, 6H), 2.02-2.15 (m, 6H), 3.26-3.34 (m, 4H), 5.63-7.70 (m, 4H)

In a reactor, 200 g (0.97 mol) of the above-prepared di-tetrahydrobenzyl ether, 0.39 g of 20% SP-D (acetic acid solution), and 669 mL of ethyl acetate were charged, followed by temperature rise up to 40°C. Next, 608 g of 29.1% peracetic acid were added dropwise over 5 hours, followed by aging for 3 hours. The organic layer was then washed sequentially with an alkaline aqueous solution three times and with ion-exchanged water two times, subjected to distillation under reduced pressure, and yielded bis(3,4-epoxycyclohexylmethyl) ether as a colorless, transparent liquid (in a yield of 77%).

Preparation Example 3: Synthesis of 2,2-bis(3,4-epoxycyclohex-1-yl)propane (a-3)

In a 1-L jacketed flask equipped with a stirrer, a condenser, a thermometer, and a nitrogen inlet tube, 36 g of water, 12.0 g of sodium hydrogensulfate, 500 g of isopropylidene-4,4'-dicyclohexanol (supplied by Aldrich), and 500 g of Solvesso 150 (supplied by Exxon Mobile Corporation) as a solvent were placed, followed by dehydration reaction at 100°C. The time point when water distillation was ceased was defined as reaction completion.

The reaction mixture was analyzed by gas chromatography to find that 2,2-bis(3,4-cyclohexenyl)propane was formed in a yield of 96%. The reaction mixture was washed with 500 mL of ion-exchanged water using a separatory funnel, the organic layer was subjected to distillation under reduced pressure, and yielded 387.0 g of 2,2-bis(3,4-cyclohexenyl)propane as a colorless, transparent liquid. This had a purity of 96.1%.

In a 1-L jacketed flask as above, 100 g of the above-prepared 2,2-bis(3,4-cyclohexenyl)propane and 30 g of ethyl acetate were placed, and 307.2 g of a solution (peracetic acid concentration: 29.1 weight percent, water content: 0.47 weight percent) of approximately anhydrous peracetic acid in ethyl acetate were added dropwise over about 2 hours while blowing nitrogen into the gas phase portion and controlling the temperature in the reaction system to be 30°C. After the completion of the dropwise addition of peracetic acid, the mixture was aged at 30°C for 3 hours, and the reaction was completed. The mixture upon the reaction completion was further washed with water at 30°C, from which low-boiling components were removed at 70°C and 20 mmHg, and yielded 99.4 g of 2,2-bis(3,4-epoxycyclohex-1-yl)propane.

The prepared product had, as properties, an oxirane oxygen content of 11.3% and a viscosity of 3550 cP (25°C). The $^1$H-NMR spectrum of the product demonstrated that a peak at a δ of about 4.5 to about 5 ppm disappeared, where this peak is assigned to an internal double bond; but a peak at a δ of about 2.9 to about 3.1 ppm appeared, where this peak is assigned to an epoxy-derived proton.

Examples 1 to 8 and Comparative Examples 1 to 5

Photocurable compositions were prepared by mixing a cycloaliphatic epoxy compound, an oxetane compound, a glycidyl ether epoxy compound, an antioxidant, an ultraviolet absorber, and an additive in proportions given in Table 1 below. The photocurable compositions according to Examples 1 to 8 and Comparative Examples 1 to 5 were evaluated for degree of swelling of the polydimethylsiloxane, viscosity, 5% weight loss and transmittance (400 nm) of the cured article, releasability upon 50th transfer, and mold surface roughness after 50 transfer operations. Results of the evaluations are given in Table 1.

Solubility Parameter Calculation Method

The solubility parameters (δ) of the compounds in Table 1 are values determined at 25°C by the Fedors' method and were calculated in accordance with the method described in Polym. Eng. Sci., 14, 147(1974). Specifically, the solubility parameters were calculated according to the following equation. The evaporation energy and the molar volume of an atom or atomic group are known typically from the literature.

[Math. 1]

$$\delta = (\Sigma e_i / \Sigma v_i)^{1/2}$$

wherein:

$e_i$ represents the evaporation energy of an atom or atomic group; and
$v_i$ represents the molar volume of the atom or atomic group.

Degree of Swelling of Polydimethylsiloxane

KE-1606 (trade name, supplied by Shin-Etsu Silicones) was cured at 60°C for 24 hours, treated at 150°C for one hour, and yielded a polydimethylsiloxane resin sheet (20 mm by 20 mm by 0.5 mm). The prepared resin sheet was weighed, and then immersed in a sample photocurable composition, and left stand at 25°C for 3 hours. The resin sheet was retrieved, from which the composition deposited on the surface was wiped off, and the resin sheet was again weighed. The degree of swelling of the polydimethylsiloxane was calculated according to the following expression:

Degree of Swelling of Polydimethylsiloxane = [(Weight after immersion in the composition) - (Weight before immersion in the composition)]/(Weight before immersion in the composition) × 100 (%)

Viscosity

The viscosities (Pa·s) of the photocurable compositions obtained in the examples and the comparative examples were measured at a temperature of 25°C and a rotation speed of 20 revolutions per second using a rheometer (trade name PHYSICA UDS200, supplied by Paar Physica).

5% Weight Loss Temperature of Cured Article

A Teflon® spacer having a length of 20 mm, a width of 20 mm, and a thickness of 0.5 mm was prepared and placed between glass slides (trade name S2111, Matsunami Glass Ind., Ltd.), where the glass slides had been subjected to surface release treatment by immersing the glass slides in OPTOOL HD1000 (trade name, supplied by Daikin Industries Ltd.) and then leaving stand in a chemical hood for 24 hours. A sample curable composition was charged into the space between the glass slides, irradiated with light using a UV-LED (at a wavelength of 365 nm) at an integral irradiance of 3000 $mJ/cm^2$ and yielded a cured article. The obtained cured article was cut out in an amount of 10 mg, on which the 5% weight loss temperature was measured under conditions as follows using a TG-DTA (trade name EXSTAR 6300, supplied by Hitachi High-Tech Science Corporation). Thus, the heat resistance of the sample was evaluated.

TG-DTA Conditions

Rate of temperature rise: 20°C/min
Atmosphere: nitrogen
Temperature range: 30°C to 400°C

Transmittance (400 nm)

A sample as with the cured article on which the 5% weight loss temperature was measured was prepared, and the transmittance (400 nm) of the cured article sample was measured. The transmittance was measured using a spectrophotometer (trade name U-3900, supplied by Hitachi High-Technologies Corporation).

Releasability upon 50th Transfer

Flat-shaped silicon molds (50 mm in diameter by 0.5 mm in thickness) were prepared by curing KE-1606 (trade name, supplied by Shin-Etsu Silicones) at 60°C for 24 hours and subsequently treating at 150°C for one hour. These were used as a cope and a drag to constitute a mold, between which spacers having a thickness t of 0.5 mm were

sandwiched. Using the mold, transfer was performed successively 50 times, and how demolding upon 50th transfer was was evaluated.

Good: the molded article could be demolded from the mold within 10 seconds;
Fair: the molded article took 10 seconds or longer to demold, but could be demolded without cracking or breakage; and
Poor: the molded article was broken upon demolding.

Mold Surface Roughness after 50 Transfer Operations

The surface roughness of the silicon mold after performing a transfer operation successively 50 times was measured using ET4000 (supplied by Kosaka Laboratory Ltd.). The surface roughness was measured at a measurement speed of 10 μm and a measurement distance of 1.25 mm, with a stylus having a tip radius of 2 pm, at a cutoff of 0.25 mm.

[Table 1]

TABLE 1

| | Compound name | Solubility parameter | Molecular weight | Example 1 | Example 2 | Example 3 | Example 4 | Example 5 | Example 6 | Example 7 | Example 8 | Comp. Ex.1 | Comp. Ex. 2 | Comp. Ex. 3 | Camp. Ex. 4 | Comp. Ex. 5 |
|---|---|---|---|---|---|---|---|---|---|---|---|---|---|---|---|---|
| Cycloaliphatic epoxy compound | (a-1) | 9.69 | | 30 | 40 | | 30 | 15 | 30 | | | | 40 | 30 | 30 | 30 |
| | (a-2) | 9.64 | | | | | | | | 30 | | | | | | |
| | (a-3) | 9.23 | | | | | | | | | 30 | | | | | |
| | CELLOXIDE 2021P | 10.26 | | 10 | | 50 | 10 | | 10 | 10 | 10 | | 20 | | 10 | |
| Oxetane compound | OXT 101 | 11.6 | | 20 | | 20 | 20 | | 20 | 20 | 20 | 30 | 20 | | | 20 |
| | OXT 221 | 8.92 | | | | | | | | | | | | 20 | 20 | |
| | OXBP | 10.3 | | | 20 | | | 35 | | | | | | | | |
| Glycidyl ether epoxy compound | YX 8000 | 9.19 | 353 | | | | | | | | | | | | 40 | |
| | YH 300 | 9.57 | 260 | | 40 | 10 | | | | | | | | | | |
| | EPOLIGHT 100E | 10.05 | 218 | | | | | | | | | | | | | 20 |
| | BPO-20E | 10.09 | 457 | | | 20 | | 50 | | | | 40 | 20 | 50 | | 30 |
| | BPO-60E | 10.34 | 561 | 40 | | | 40 | | 40 | 40 | 40 | 30 | | | | |
| Photoinitiator | CPI-101A | | | 0.5 | 0.5 | 0.5 | | | 0.5 | 0.5 | 0.5 | 0.5 | 0.5 | 0.5 | 0.5 | 0.5 |
| | (d-1) | | | | | | 0.5 | 0.5 | | | | | | | | |
| Antioxidant | IN1010 | | | 1 | 1 | 1 | 1 | 1 | 1 | 1 | 1 | 1 | 1 | 1 | 1 | 1 |
| UV absorber | KEMISORB 79 | | | | | | | | 0.05 | | | | | | | |
| Additive | BYK 333 | | | 0.1 | 0.1 | 0.1 | 0.1 | 0.1 | 0.1 | 0.1 | 0.1 | 0.1 | 0.1 | 0.1 | 0.1 | 0.1 |
| Degree of swelling of polydimethylsiloxane | | | | 2.5% | 2.8% | 1.4% | 2.5% | 1.1% | 2.5% | 2.0% | 3.0% | 0.9% | 3.2% | 4.6% | 5.8% | 4.0% |
| Physical properties | Viscosity (Pa·s) | | | 0.17 | *0.20* | 0.16 | 0.17 | 1.9 | 0.17 | 0.17 | 0.46 | 1.4 | 0.11 | 0.27 | 0.14 | **0.28** |

(continued)

| | | Compound name | Solubility parameter | Molecular weight | Example 1 | Example 2 | Example 3 | Example 4 | Example 5 | Example 6 | Example 7 | Example 8 | Comp. Ex.1 | Comp. Ex. 2 | Comp. Ex. 3 | Camp. Ex. 4 | Comp. Ex. 5 |
|---|---|---|---|---|---|---|---|---|---|---|---|---|---|---|---|---|---|
| Curability | 5% Weight loss temperature (°C) | | | | 377 | 372 | 366 | 360 | 372 | 367 | 375 | 389 | Not cured | 380 | 379 | 385 | 359 |
| Optical properties | Transmittance (400 nm) (%) | | | | 903 | 87.3 | 90.4 | 89.6 | 80.3 | 89.3 | 89.4 | 89.0 | | 88.5 | 87.8 | 90.7 | 88.9 |
| Moldability | Releasability upon 50th transfer | | | | Good | Good | Good | Good | Good | Good | Good | Good | | Fair | Poor | Poor | Fair |
| | Mold surface roughness after 50 transfer operations (nm) | | | | 172 | 197 | 161 | 175 | 156 | 176 | 165 | 186 | | 199 | 250 | 267 | 254 |

The abbreviations in Table 1 designate as follows:

(a-1): the compound prepared in Preparation Example 1, (3,4,3',4'-diepoxy)bicyclohexyl
(a-2): the compound prepared in Preparation Example 2, bis(3,4-epoxycyclohexylmethyl) ether
(a-3): the compound prepared in Preparation Example 3, 2,2-bis(3,4-epoxycyclohex-1-yl)propane
CELLOXIDE 2021P: 3,4-epoxycyclohexylmethyl (3,4-epoxy)cyclohexanecarboxylate, CELLOXIDE 2021P (trade name, supplied by Daicel Corporation)
OXT101: 3-ethyl-3-hydroxymethyloxetane, ARON OXETANE OXT-101 (trade name, supplied by Toagosei Co., Ltd.)
OXT221: bis[1-ethyl(3-oxetanyl)]methyl ether, ARON OXETANE OXT-221 (trade name, supplied by Toagosei Co., Ltd.)
OXBP: 4,4'-bis[(3-ethyl-3-oxetanyl)methoxymethyl]biphenyl, OXBP (trade name, supplied by Ube Industries, Ltd.)
YX8000: a hydrogenated bisphenol-A epoxy resin, YX8000 (trade name, supplied by Mitsubishi Chemical Corporation)
YH300: an aliphatic glycidyl ether epoxy compound, YH-300 (trade name, supplied by NIPPON STEEL & SUMIKIN CHEMICAL CO., LTD.)
EPOLIGHT 100E: an aliphatic glycidyl ether epoxy compound, EPOLIGHT 100E (trade name, supplied by Kyoeisha Chemical Co., Ltd.)
BPO-20E: bisphenol-A diglycidyl ether, Rikaresin BPO-20E (trade name, supplied by New Japan Chemical Co., Ltd.)
BPO-60E: bisphenol-A diglycidyl ether, Rikaresin BPO-60E (trade name, supplied by New Japan Chemical Co., Ltd.)
CPI-101A: a photoinitiator (photoacid generator), CPI-101A (trade name, supplied by San-Apro Ltd.)
(d-1): 4-(phenylthio)phenyldiphenylsulfonium phenyltris(pentafluorophenyl)borate
IN1010: tetrakis[3-(3,5-di-t-butyl-4-hydroxyphenyl propionate), Irganox 1010 (trade name, supplied by BASF SE)
KEMISORB 79: a benzotriazole ultraviolet absorber, KEMISORB 79 (trade name, supplied by Chemipro Kasei Kaisha, Ltd.)
BYK 333: a polyether-modified polydimethylsiloxane (silicon-containing surface conditioner), BYK-333 (trade name, supplied by BYK Japan KK)

The data in Table 1 demonstrate that the photocurable compositions according to the present invention have curability and viscosity properties at excellent levels, still less cause swelling of a polydimethylsiloxane, and offer excellent releasability from a silicon mold even in repeated use. The photocurable compositions according to the present invention therefore allow the silicon mold to maintain satisfactory durability and to be usable repeatedly, and also have excellent economic efficiency.

Industrial Applicability

The photocurable compositions according to the present invention are used in a UV imprint molding step in production of optical components, where the UV imprint molding step is the step of applying ultraviolet rays to the photocurable compositions in a mold to perform successive molding (transfer). The cured articles and optical components according to the present invention are used typically as flash lenses and Fresnel lenses.

## Claims

1. A photocurable composition comprising components (A'), (B), (C), and (D),

    the component (A') being present in a content of 10 to 50 weight percent of the totality of photocurable compounds contained in the photocurable composition,
    the component (C) being present in a content of 10 to 60 weight percent of the totality of photocurable compounds contained in the photocurable composition,
    the component (A') being at least one selected from the group consisting of 1,2-epoxy-1,2-bis(3,4-epoxycyclohex-1-yl)ethane, 2,2-bis(3,4-epoxycyclohex-1-yl)propane, and 1,2-bis(3,4-epoxycyclohex-1-yl)ethane,
    the component (B) being an oxetane compound having a solubility parameter of 9.5 $(cal/cm^3)^{1/2}$ or more as determined by Fedors' method,
    the component (C) being a glycidyl ether epoxy compound having a molecular weight of 250 or more, and
    the component (D) being a photoinitiator.

2. A photocurable composition comprising components (A), (B), (C'), and (D),

the component (A) being present in a content of 10 to 50 weight percent of the totality of photocurable compounds contained in the photocurable composition,
the component (A) being a cycloaliphatic epoxy compound represented by Formula (a):

[Chem. 1]

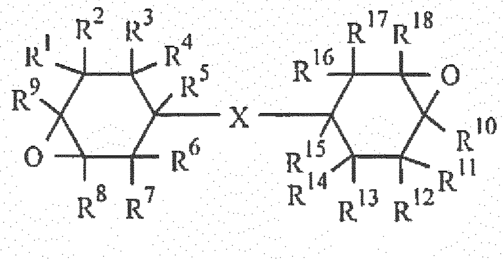

(a)

wherein $R^1$ to $R^{18}$ are each, identically or differently, selected from hydrogen, halogen, a hydrocarbon group optionally containing oxygen or halogen, and optionally substituted alkoxy; and X is selected from a single bond and a linkage group,
the component (B) being an oxetane compound having a solubility parameter of 9.5 $(cal/cm^3)^{1/2}$ or more as determined by Fedors' method,
the component (C') being a glycidyl ether epoxy compound having a molecular weight of 250 or more, wherein the glycidyl ether epoxy compound is a alicyclic glycidyl ether epoxy compound or aliphatic glycidyl ether epoxy compound, and
the component (D) being a photoinitiator.

**3.** The photocurable composition according to one of claims 1 and 2,
wherein the component (B) is present in a content of 10 to 60 weight percent of the totality of photocurable compounds contained in the photocurable composition.

**4.** The photocurable composition according to claim 2,
wherein the component (C') is present in a content of 10 to 60 weight percent of the totality of photocurable compounds contained in the photocurable composition.

**5.** The photocurable composition according to any one of claims 1 to 4,
wherein the component (D) is present in a proportion of 0.05 to 10 parts by weight per 100 parts by weight of the totality of photocurable compounds contained in the photocurable composition.

**6.** The photocurable composition according to any one of claims 1 to 5,
wherein the component (A) and (A') have a solubility parameter of 9.2 $(cal/cm^3)^{1/2}$ or more as determined by the Fedors' method.

**7.** The photocurable composition according to any one of claims 1 to 6,
wherein the component (C) and (C') have a solubility parameter of 9.5 $(cal/cm^3)^{1/2}$ or more as determined by the Fedors' method.

**8.** The photocurable composition according to any one of claims 1 to 7,
further comprising at least one of an antioxidant and an ultraviolet absorber.

**9.** A cured article resulting from curing the photocurable composition according to any one of claims 1 to 8.

**10.** An optical component comprising
the cured article according to claim 9.

**11.** An optical device comprising

the optical component according to claim 10.

**12.** A method for producing a cured article, the method comprising the steps of:

1) charging a following photocurable composition into an optical-component mold; and

a photocurable composition: the photocurable composition comprising components (A'), (B), (C), and (D),
the component (A') being present in a content of 10 to 50 weight percent of the totality of photocurable compounds contained in the photocurable composition,
the component (C) being present in a content of 10 to 60 weight percent of the totality of photocurable compounds contained in the photocurable composition,
the component (A') being at least one selected from the group consisting of 1,2-epoxy-1,2-bis(3,4-epoxycyclohex-1-yl)ethane, 2,2-bis(3,4-epoxycyclohex-1-yl)propane, and 1,2-bis(3,4-epoxycyclohex-1-yl)ethane,
the component (B) being an oxetane compound having a solubility parameter of 9.5 $(cal/cm^3)^{1/2}$ or more as determined by Fedors' method,
the component (C) being a glycidyl ether epoxy compound having a molecular weight of 250 or more, and
the component (D) being a photoinitiator,

2) irradiating the curable composition with light to cure the curable composition.

**13.** The method according to claim 12 for producing a cured article,
wherein the optical-component mold is a silicon mold.

**14.** The method according to claim 13 for producing a cured article,
wherein the silicon mold is made from a polydimethylsiloxane.

**15.** The method according to any one of claims 12 to 14 for producing a cured article,
wherein the step 2) comprises applying light at 350 to 450 nm from a UV-LED to cure the curable composition.

**Patentansprüche**

**1.** Photohärtbare Zusammensetzung, umfassend die Komponenten (A'), (B), (C) und (D),

wobei die Komponente (A') in einem Gehalt von 10 bis 50 Gewichtsprozent der Gesamtheit der in der photohärtbaren Zusammensetzung enthaltenen photohärtbaren Verbindungen vorhanden ist,
wobei die Komponente (C) in einem Gehalt von 10 bis 60 Gewichtsprozent der Gesamtheit der in der photohärtbaren Zusammensetzung enthaltenen photohärtbaren Verbindungen vorhanden ist,
wobei es sich bei der Komponente (A') um mindestens eine handelt, ausgewählt aus der Gruppe, bestehend aus 1,2-Epoxy-1,2-bis(3,4-epoxycyclohex-1-yl)ethan, 2,2-Bis(3,4-epoxycyclohex-1-yl)propan und 1,2-Bis(3,4-epoxycyclohex-1-yl)ethan,
wobei es sich bei der Komponente (B) um eine Oxetanverbindung mit einem Löslichkeitsparameter von 9,5 $(cal/cm^3)^{1/2}$ oder mehr, bestimmt nach dem Verfahren von Fedors, handelt,
wobei es sich bei der Komponente (C) um eine Glycidyletherepoxyverbindung mit einem Molekulargewicht von 250 oder mehr handelt und
wobei es sich bei der Komponente (D) um einen Photoinitiator handelt.

**2.** Photohärtbare Zusammensetzung, umfassend die Komponenten (A), (B), (C') und (D),

wobei die Komponente (A) in einem Gehalt von 10 bis 50 Gewichtsprozent der Gesamtheit der in der photohärtbaren Zusammensetzung enthaltenen photohärtbaren Verbindungen vorhanden ist,
wobei es sich bei der Komponente (A) um eine cycloaliphatische Epoxyverbindung handelt, dargestellt durch Formel (a):

[Chem. 1]

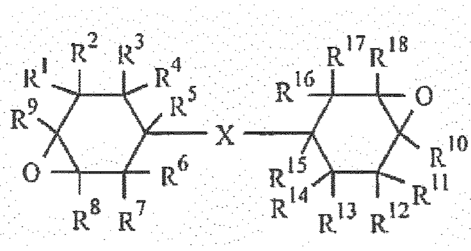

(a)

wobei $R^1$ bis $R^{18}$ jeweils gleich oder unterschiedlich ausgewählt sind aus Wasserstoff, Halogen, einer Kohlenwasserstoffgruppe, die gegebenenfalls Sauerstoff oder Halogen enthält, und gegebenenfalls substituiertem Alkoxy; und X ausgewählt ist aus einer Einfachbindung und einer Verbindungsgruppe,
wobei es sich bei der Komponente (B) um eine Oxetanverbindung mit einem Löslichkeitsparameter von 9,5 $(cal/cm^3)^{1/2}$ oder mehr, bestimmt nach dem Verfahren von Fedors, handelt,
wobei es sich bei der Komponente (C') um eine Glycidyletherepoxyverbindung mit einem Molekulargewicht von 250 oder mehr handelt und
wobei es sich bei der Glycidyletherepoxyverbindung um eine alicyclische Glycidyletherepoxyverbindung oder eine aliphatische Glycidyletherepoxyverbindung handelt und
wobei es sich bei der Komponente (D) um einen Photoinitiator handelt.

**3.** Photohärtbare Zusammensetzung gemäß einem der Ansprüche 1 und 2,
wobei die Komponente (B) in einem Gehalt von 10 bis 60 Gewichtsprozent der Gesamtheit der in der photohärtbaren Zusammensetzung enthaltenen photohärtbaren Verbindungen vorhanden ist.

**4.** Photohärtbare Zusammensetzung gemäß Anspruch 2,
wobei die Komponente (C') in einem Gehalt von 10 bis 60 Gewichtsprozent der Gesamtheit der in der photohärtbaren Zusammensetzung enthaltenen photohärtbaren Verbindungen vorhanden ist.

**5.** Photohärtbare Zusammensetzung gemäß einem der Ansprüche 1 bis 4,
wobei die Komponente (D) in einem Anteil von 0,05 bis 10 Gewichtsteilen pro 100 Gewichtsteilen der Gesamtheit der in der photohärtbaren Zusammensetzung enthaltenen photohärtbaren Verbindungen vorhanden ist.

**6.** Photohärtbare Zusammensetzung gemäß einem der Ansprüche 1 bis 5,
wobei die Komponenten (A) und (A') einen Löslichkeitsparameter von 9,2 $(cal/cm^3)^{1/2}$ oder mehr, bestimmt nach dem Verfahren von Fedors, aufweisen.

**7.** Photohärtbare Zusammensetzung gemäß einem der Ansprüche 1 bis 6,
wobei die Komponenten (C) und (C') einen Löslichkeitsparameter von 9,5 $(cal/cm^3)^{1/2}$ oder mehr, bestimmt nach dem Verfahren von Fedors, aufweisen.

**8.** Photohärtbare Zusammensetzung gemäß einem der Ansprüche 1 bis 7,
ferner umfassend mindestens eines aus einem Antioxidans und einem UV-Absorptionsmittel.

**9.** Gehärteter Gegenstand, resultierend aus dem Härten der photohärtbaren Zusammensetzung gemäß einem der Ansprüche 1 bis 8.

**10.** Optische Komponente, umfassend
den gehärteten Gegenstand gemäß Anspruch 9.

**11.** Optische Vorrichtung, umfassend
die optische Komponente gemäß Anspruch 10.

**12.** Verfahren zum Herstellen eines gehärteten Gegenstands, wobei das Verfahren die Schritte umfasst:

1) Einfüllen einer folgenden photohärtbaren Zusammensetzung in eine Form für optische Komponenten; und

eine photohärtbare Zusammensetzung: wobei die photohärtbare Zusammensetzung die Komponenten (A'), (B), (C) und (D) umfasst,
wobei die Komponente (A') in einem Gehalt von 10 bis 50 Gewichtsprozent der Gesamtheit der in der photohärtbaren Zusammensetzung enthaltenen photohärtbaren Verbindungen vorhanden ist,
wobei die Komponente (C) in einem Gehalt von 10 bis 60 Gewichtsprozent der Gesamtheit der in der photohärtbaren Zusammensetzung enthaltenen photohärtbaren Verbindungen vorhanden ist,
wobei es sich bei der Komponente (A') um mindestens eine handelt, ausgewählt aus der Gruppe, bestehend aus 1,2-Epoxy-1,2-bis(3,4-epoxycyclohex-1-yl)ethan, 2,2-Bis(3,4-epoxycyclohex-1-yl)propan und 1,2-Bis(3,4-epoxycyclohex-1-yl)ethan,
wobei es sich bei der Komponente (B) um eine Oxetanverbindung mit einem Löslichkeitsparameter von 9,5 $(cal/cm^3)^{1/2}$ oder mehr, bestimmt nach dem Verfahren von Fedors, handelt,
wobei es sich bei der Komponente (C) um eine Glycidyletherepoxyverbindung mit einem Molekulargewicht von 250 oder mehr handelt und
wobei es sich bei der Komponente (D) um einen Photoinitiator handelt,

2) Bestrahlen der härtbaren Zusammensetzung mit Licht, um die härtbare Zusammensetzung zu härten.

**13.** Verfahren gemäß Anspruch 12 zum Herstellen eines gehärteten Gegenstands, wobei die Form für optische Komponenten eine Silikonform ist.

**14.** Verfahren gemäß Anspruch 13 zum Herstellen eines gehärteten Gegenstands, wobei die Silikonform aus einem Polydimethylsiloxan hergestellt ist.

**15.** Verfahren gemäß einem der Ansprüche 12 bis 14 zum Herstellen eines gehärteten Gegenstands,
wobei der Schritt 2) das Anwenden von Licht bei 350 bis 450 nm von einer UV-LED umfasst, um die härtbare Zusammensetzung zu härten.

## Revendications

**1.** Composition photodurcissable comprenant des constituants (A'), (B), (C) et (D),

le constituant (A') étant présent à hauteur de 10 à 50 pourcent en poids de la totalité des composés photodurcissables contenus par la composition photodurcissable,
le constituant (C) étant présent à hauteur de 10 à 60 pourcent en poids de la totalité des composés photodurcissables contenus par la composition photodurcissable,
le constituant (A') étant au moins un composé choisi dans le groupe constitué par le 1,2-époxy-1,2-bis(3,4-époxycyclohex-1-yl)éthane, le 2,2-bis(3,4-époxycyclohex-1-yl)propane et le 1,2-bis(3,4-époxycyclohex-1-yl)éthane,
le constituant (B) étant un composé oxétane ayant un paramètre de solubilité de 9,5 $(cal/cm^3)^{1/2}$ ou plus, ainsi que déterminé par la méthode de Fedors,
le constituant (C) étant un composé époxy éther de glycidyle ayant une masse moléculaire de 250 ou plus, et
le constituant (D) étant un photoinitiateur.

**2.** Composition photodurcissable comprenant des constituants (A), (B), (C') et (D),

le constituant (A) étant présent à hauteur de 10 à 50 pourcent en poids de la totalité des composés photodurcissables contenus par la composition photodurcissable,
le constituant (A) étant un composé époxy cycloaliphatique représenté par la formule (a) :

[Substance chimique 1]

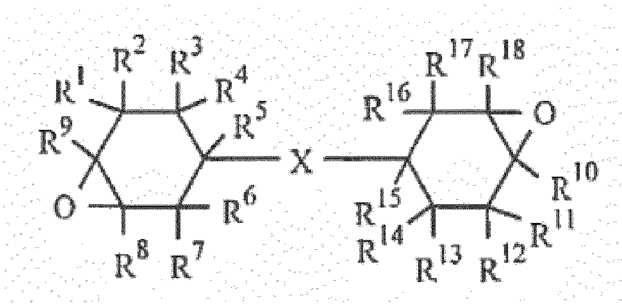
(a)

dans laquelle les $R^1$ à $R^{18}$ sont chacun, de façon identique ou différente, choisis parmi un hydrogène, un halogène, un groupe hydrocarbure contenant éventuellement de l'oxygène ou de l'halogène, et un alcoxy éventuellement substitué ; et X est choisi parmi une liaison simple et un groupe de liaison,
le constituant (B) étant un composé oxétane ayant un paramètre de solubilité de 9,5 $(cal/cm^3)^{1/2}$ ou plus, ainsi que déterminé par la méthode de Fedors,
le constituant (C') étant un composé époxy éther de glycidyle ayant une masse moléculaire de 250 ou plus,
le composé époxy éther de glycidyle étant un composé époxy éther de glycidyle alicyclique ou un composé époxy éther de glycidyle aliphatique, et
le constituant (D) étant un photoinitiateur.

**3.** Composition photodurcissable selon l'une des revendications 1 et 2,
dans laquelle le constituant (B) est présent à hauteur de 10 à 60 pourcent en poids de la totalité des composés photodurcissables contenus par la composition photodurcissable.

**4.** Composition photodurcissable selon la revendication 2,
dans laquelle le constituant (C') est présent à hauteur de 10 à 60 pourcent en poids de la totalité des composés photodurcissables contenus par la composition photodurcissable.

**5.** Composition photodurcissable selon l'une quelconque des revendications 1 à 4,
dans laquelle le constituant (D) est présent dans une proportion de 0,05 à 10 parties en poids pour 100 parties en poids de la totalité des composés photodurcissables contenus par la composition photodurcissable.

**6.** Composition photodurcissable selon l'une quelconque des revendications 1 à 5,
dans laquelle les constituants (A) et (A') ont un paramètre de solubilité de 9,2 $(cal/cm^3)^{1/2}$ ou plus, ainsi que déterminé par la méthode de Fedors.

**7.** Composition photodurcissable selon l'une quelconque des revendications 1 à 6,
dans laquelle les constituants (C) et (C') ont un paramètre de solubilité de 9,5 $(cal/cm^3)^{1/2}$ ou plus, ainsi que déterminé par la méthode de Fedors.

**8.** Composition photodurcissable selon l'une quelconque des revendications 1 à 7,
comprenant en outre un antioxydant et/ou un absorbeur d'ultraviolet.

**9.** Article durci résultant du durcissement de la composition photodurcissable selon l'une quelconque des revendications 1 à 8.

**10.** Composant optique comprenant l'article durci selon la revendication 9.

**11.** Dispositif optique comprenant le composant optique selon la revendication 10.

**12.** Procédé de production d'un article durci, le procédé comprenant les étapes consistant :

1) à introduire une composition photodurcissable ci-dessous dans un moule pour composant optique ; et

composition photodurcissable : la composition photodurcissable comprenant des constituants (A'), (B), (C) et (D),
le constituant (A') étant présent à hauteur de 10 à 50 pourcent en poids de la totalité des composés

photodurcissables contenus par la composition photodurcissable,
le constituant (C) étant présent à hauteur de 10 à 60 pourcent en poids de la totalité des composés photodurcissables contenus par la composition photodurcissable,
le constituant (A') étant au moins un composé choisi dans le groupe constitué par le 1,2-époxy-1,2-bis(3,4-époxycyclohex-1-yl)éthane, le 2,2-bis(3,4-époxycyclohex-1-yl)propane et le 1,2-bis(3,4-époxycyclohex-1-yl)éthane,
le constituant (B) étant un composé oxétane ayant un paramètre de solubilité de 9,5 $(cal/cm^3)^{1/2}$ ou plus, ainsi que déterminé par la méthode de Fedors,
le constituant (C) étant un composé époxy éther de glycidyle ayant une masse moléculaire de 250 ou plus, et
le constituant (D) étant un photoinitiateur,

2) à irradier la composition durcissable avec de la lumière pour durcir la composition durcissable.

**13.** Procédé selon la revendication 12 pour produire un article durci,
le moule pour composant optique étant un moule en silicone.

**14.** Procédé selon la revendication 13 pour produire un article durci,
le moule en silicone étant constitué d'un polydiméthylsiloxane.

**15.** Procédé selon l'une quelconque des revendications 12 à 14 pour produire un article durci,
dans lequel l'étape 2) comprend l'application d'une lumière de 350 à 450 nm d'une LED UV pour durcir la composition durcissable.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2015107580 A
- JP 2015139675 A
- JP 2005119124 A
- JP 4124991 B
- JP H11092539 A
- JP 4800383 B
- JP 3671658 B
- WO 2014112295 A1
- US 2010119835 A1
- JP 2014038349 A
- JP 2012128106 A
- JP 2013137442 A
- JP H04127101 A
- JP 2002264140 A
- JP 2610029 B
- JP H09141663 A
- JP H0611769 A